# EUROPEAN PATENT APPLICATION

(11) **EP 3 863 298 A1**
(43) Date of publication of application: **11.08.2021**
(21) Application number: 20155401.1
(22) Date of filing: 04.02.2020
(51) Int. Cl.: H04R 1/10, H04R 25/00, A61F 11/08

(54) **PROFILED EAR PIECE AND DEVICE FOR THE GENERATION OF AN EAR PIECE**

(71) Applicant: PRO3DURE MEDICAL GMBH, 44227 Dortmund (DE)
(72) Inventor: SCHWAN, Axel, 99089 Erfurt (DE); KLARE, Martin, 44227 Dortmund (DE)
(74) Representative: Schulz Junghans Patentanwälte PartGmbB

(57) **Abstract**

The invention is related to a casting form (1) for the generation of an ear piece (100) or a part of an ear piece (100), wherein the ear piece (100) comprises a contact section (120) and a non-contact section (110). The casting form (1) comprises an inner surface (10) delimiting an inner volume (12) for receiving a casting material, wherein the inner volume (12) is at least a part of the contact section (120). The inner surface (10) comprises at least one profiled section (20) comprising at least one texturing element (30). The invention is further related to a method for generating a casting form (1). The invention further comprises an ear piece (100).

## Description

The invention is related to an ear piece, a casting form for the generation of an ear piece and a method for the generation of a casting form.

In very different contexts, ear pieces are used. For instance, an ear piece is an earplug that can be used for noise protection (e.g. for construction workers). Another embodiment of an earplug can be used by musicians for noise control and/or to filter particular frequencies. Also a hearing aid can comprise an ear piece that is inserted in the ear, in particular the ear canal of the user. Yet another embodiment of an ear piece is an earbud of a head-phone.

Independent of the particular function of the ear piece, these ear pieces have in common that they are at least partially inserted into the ear, in particular in the ear canal, of a user when used as intended. To fulfil the function properly, the ear piece has to fit adequately and to remain the position even in case the user moves. Moreover, often, an ear piece is used over long periods of time. Therefore, an ear piece has to be comfortable to wear.

Hence, there is a need to provide an ear piece that both has a good grip and is comfortable to wear. Moreover, a device is of interest for an easy and inexpensive generation of such an ear piece, as well as a method for the generation of such a device.

This is solved by the casting form according to claim 1, the method for the generation of the casting form described in claim 5 as well as the ear piece according to claim 6. Embodiments of the aspects of the present invention are stated in the corresponding sub claims and are described below.

A first aspect of the invention is related to a casting form for the generation of an ear piece or a part of an ear piece, wherein the ear piece comprises a contact section and a non-contact section. The casting form comprises an inner surface delimiting an inner volume for receiving a casting material, wherein the inner volume is at least a part of the contact section, and wherein the inner surface comprises at least one profiled section comprising at least one texturing element.

In context of this application the non-contact section of an ear piece can be the part of the ear piece that is not inserted in the ear of a user when the ear piece is in use as intended. The non-contact section can comprise a faceplate.

The contact section can be the section of the ear piece that is inserted in the ear, in particular in the ear canal, when the ear piece is in use as intended. At least a region (or a subsection) of the contact section can be in contact with the ear of the user (particularly the user's ear canal), when the ear piece is inserted in the user's ear as intended. In an embodiment, the contact section and the non-contact section adjoin each other.

The profiled section can comprise one texturing element. According to an alternative embodiment, the profiled section comprises a plurality of texturing elements. The profiled section can have a texture, i.e. a structure. The profiled section can have a texture surface.

According to an embodiment, the casting form can comprise a plurality of profiled sections.

In an embodiment in that the casting form is configured for the generation of an ear piece, it is used for the generation of the entire ear piece. Advantageously, it can be used several times as a template for the generation of an ear piece. The ear piece can easily be generated by filling in a material into the casting form.

In an embodiment in that the casting form is configured for the generation of a part of an ear piece, this casting form can be inserted into a supporting form for the generation of the entire ear piece. The supporting form together with the inserted casting form can be used as a template for the generation of an ear piece. The casting form can easily be inserted into the supporting form. In an embodiment, the casting form can easily be removed from the supporting form. Hence, advantageously, a fast and easy replacement of the profiled section can be achieved. Hence, successively, different casting forms (in particular with different profiled sections) can be inserted in the same supporting form such that it can be used for the generation of different ear pieces.

According to an embodiment, a plurality of casting forms for the generation of a part of an ear piece can be combined to provide a plurality of templates for the generation of an ear piece or that can be inserted into a supporting form.

An individual casting form for the generation of a part of an ear piece can have a particular profiled section. By combining different combinations of casting forms for the generation of a part (with different profiled sections), various templates can be provided without the need to provide a specific casting form for each possible combination of profiled structures. This advantageously increases the variability and decreases the costs.

In an embodiment, the inner volume extends along a central axis, wherein the at least one texturing element protrudes towards the central axis into the inner volume.

In other words this means that the texturing element can recess the inner volume. In the context of the application this is also referred to as texturing element that is "directed inwards".

In an alternative embodiment, the inner volume extends along a central axis, wherein the at least one texturing element protrudes away from the central axis.

In other words this means that the texturing element can stick out of the inner volume, i.e. the texturing element is "directed outwards".

According to an embodiment, the at least one profiled section comprises a plurality of texturing elements. A texturing element can be directed outwards or inwards. According to an embodiment, each texturing element of the plurality of texturing elements has the same orientation. In an embodiment, each texturing element of the plurality of texturing elements protrudes towards the central axis into the inner volume. In another embodiment, each texturing element of the plurality of texturing elements protrudes away from the central axis. This means that all texturing elements of the plurality of texturing elements can be directed outwards.

According to an alternative embodiment, one texturing element of the plurality of texturing elements protrudes towards the central axis into the inner volume and one other texturing element of the plurality of texturing elements protrudes away the central axis. One texturing element can be directed outwards while the one other texturing element can be directed inwards. In other words this means that the one texturing element and the one other texturing element of the plurality of texturing elements can have different orientations.

According to an embodiment, the at least one texturing element has a height and/or a width and/or a length of at least 25 µm.

The length can indicate the extension of the texturing element parallel to the central axis.

The height can indicate the extension of the texturing element perpendicular to the central axis of the inner volume. In particular, the height can be the distance from a foot of the texturing element to a head of the texturing element (perpendicular to the central axis of the inner volume). At the foot of the texturing element, the texturing element arises from the inner surface. The head is distant to the inner surface.

The width can indicate the extension of the texturing element along the circumferential direction of the inner volume.

In an embodiment, two neighbouring texturing elements are distant to each other. The space between the two neighbouring texturing elements can be at least 25 µm. In particular, the space between the feet of the two neighbouring texturing elements can be at least 25 µm. In an embodiment, a space between two neighbouring texturing elements exists, wherein the two texturing elements have the same orientation (i.e. that both protrudes towards or that both protrudes away from the central axis).

According to another embodiment, two neighbouring texturing elements adjoin each other. In particular this means that there is no space between these two neighbouring texturing elements. In an embodiment, two neighbouring texturing elements that have different orientations adjoin each other. In other words this means that an inwards directed texturing element can adjoin a neighbouring outwards directed texturing element.

In an embodiment, the at least one profiled section comprises or consists of a material for 3D printing, in particular a cured photoreactive material for 3D printing.

In an embodiment, the photoreactive material for 3D printing consists of or comprises a 3D printing resin, in particular a polymer resin, in particular a photopolymer resin. According to an embodiment, the material for 3D printing is a silicone acrylate. In an embodiment, the material for 3D printing is an acrylonitrile butadiene styrene (ABS). In an alternative embodiment, the material for 3D printing is corn starch.

In an embodiment, the material can be cured in a light-dependent manner. Particularly, the material for 3D printing is a photoreactive material.

Another aspect of the invention is related to a method for the generation of a casting form for the generation of an ear piece or a part of an ear piece according to the invention, comprising the step of
- printing at least the at least one profiled section by a 3D printer, in particular a printer configured for image projection-based printing or laser-based printing.

According to an embodiment, the casting form for the generation of an ear piece or the part of an ear piece is printed by a printer configured for digital light processing (DLP) printing. In an alternative embodiment, the casting form is generated by a printer configured for laser-based stereolithography (SLA) printing. The casting form can be generated by a printer configured for LCD printing. In an embodiment, the printer is an OLED-based printer. According to an embodiment, the casting form can be generated via a fused deposition modelling (FDM) method.

The advantage of the generation of a casting form by 3D printing is that the casting form can be generated in a fast, easy and cost-effective manner. Furthermore, the 3D printer is configured such that it can print the casting form in high resolution. In particular, the profiled section can be printed in high resolution such that the texturing element or each texturing element of the plurality of texturing elements is printed precisely. In particular, a 3D printer configured for DLP or SLA printing can be used to print the profiled section in high resolution.

A further aspect of the invention is related to an ear piece for positioning in an ear of a person. The ear piece comprises a non-contact section and a contact section, in particular generated by use of the casting form for the generation of an ear piece or a part of an ear piece according to the invention.

The ear piece can comprise an outer surface that delimits a volume. In an embodiment, the contact section comprises a central axis. The contact section can extend along an extension direction. In an embodiment, the ear piece extends along a central axis.

According to an embodiment, the contact section comprises at least one structured section comprising at least one structure element.

In an embodiment, the at least one structure element is complementary to the texturing element of the casting form. In other words this means that the at least one structure element can be a negative of the texturing element of the casting form. In the context of this application, the specific texturing element that is the negative of the respective structure element is also referred to as "associated texturing element".

In an embodiment, the at least one structured section is complementary to the profiled section of the casting form. In other words this means that the at least one structured section can be a negative of the profiled section of the casting form.

The structure element can have a specific shape. The structure element can have a height, a width and/or a length. These can be determined by the respective characteristics of the associated texturing element of the casting form, in particular the shape, the height, the width and/or the length of the associated texturing element, respectively.

The structure element can be directed inwards. In other words this means that the structured element can protrude towards the central axis of the contact section (or the ear piece). This means that the structured element can recess the volume.

According to the invention, the structure element can be directed outwards. In other words this means that the structured element can protrude away from the central axis of the contact section (or the ear piece). This means that the structured element can stick out of the volume.

The orientation of the structure element, i.e. whether it recesses the volume or whether it sticks out of the volume, can be determined by the orientation of the associated texturing element.

The structured section can comprise a plurality of structure elements. According to an embodiment, each structure element of the plurality of structure elements has the same orientation, i.e. either each structure element is directed inwards or each structure element is directed outwards. In an alternative embodiment, at least one structure element of the plurality of structure elements has an orientation that differs from the orientation of at least one other structure element of the plurality of structure elements. This means that at least one structure element can be directed outwards and at least one other structure element is directed inwards.

In an embodiment, each structure element of the plurality of structure elements is equal. In particular, each structure element of the structured section has the same shape, the same height, the same width, the same length and the same orientation.

In an alternative embodiment, at least one structure element of the plurality of structure elements differs in at least one property, such as the shape, the height, the width, the length, and the orientation, from at least one other structure element of the plurality of structure elements.

In an embodiment, the distance between each two neighbouring structure elements of the plurality of structure elements is equal. In other words this means that the structure elements can be evenly distributed.

In an alternative embodiment, the structure elements are scattered. In particular, the distance between two neighbouring structure elements differs from the distance between two other neighbouring structure elements.

The structure element can be configured to achieve at least one of a plurality of functions. In an embodiment, the structure section is configured to achieve at least one of a plurality of functions. In particular, the arrangement of individual structure elements to each other, the orientation of the individual structure elements, the size (height, width, and/or length) of each of these structure elements and the shape of each structure element of the plurality of structure elements can determine the particular function of the structured section.

According to an embodiment, the structure element and/or the structured section is configured such that it increases the convenience of the user when the ear piece is positioned in the user's ear as intended. In an embodiment, the structured section is configured to decrease the pressure exerted by the ear piece on the user's ear (when used as intended) in comparison to an ear piece according to prior art. According to an embodiment, the structured section comprises a plurality of lamellas. The plurality of lamellas can increase the convenience of the user when the ear piece is inserted in the user's ear as intended.

According to an embodiment, the structure element and/or the structured section is configured to increase the holding of the ear piece in the ear of the user. In other words this means that the structure element and/or the structured section is configured to increase the grip of the ear piece when used as intended. In an embodiment, the structure element and/or the structured section is configured to decrease a shifting of the ear piece in the user's ear (when used as intended). In particular, an arbitrary shifting of the ear piece can be prevented.

In an embodiment, the structure element and/or the structures section is configured to improve the ventilation of the ear when the ear piece is inserted into the user's ear as intended.

In an embodiment, the structure element and/or the structures section is configured to improve the sealing when the ear piece is used as intended. According to an embodiment, the structure element and/or the structures section is configured for noise control (when the ear piece is used as intended). In an embodiment, the structure element and/or the structures section is configured to decrease a water intake into the ear of the user when the ear piece is used as intended.

The structure element and/or the structures section can be configured to prevent an acoustic feedback, when the ear piece is used as intended.

In an alternative embodiment, the contact section and the non-contact section each comprise at least one structured section each comprising at least one structure element.

The structure element of the contact section can differ from the structure element of the non-contact section. According to an embodiment, the structured section of the contact section can differ from the structured section of the non-contact section.

In an alternative embodiment, the structure elements of the non-contact section are equal to the structure elements comprised in the structured section of the contact section.

In an embodiment, the structured section of the non-contact section is characterised by a convenient haptics.

The structured section of the non-contact section can mimic a topology or a relief, in particular a natural topology or a natural relief, respectively.

In an embodiment, the non-contact section comprises at least one structured section comprising at least one structure element, wherein the non-contact section consists of one piece and comprises or consists of a silicone or a polyurethane.

A silicone is a polymeric compound containing silicon atoms and oxygen atoms. Additionally, a silicone can comprise carbon atoms and/or hydrogen atoms.

The silicone can be an addition-curing silicone. An addition curing silicone vulcanises, i.e. crosslinks, without the release of cleavage products. The advantage of such a silicone is that it hardly shrinks after and/or during the crosslinking process. This implies that it almost remains its size. In particular, the silicone remains its size and shape. The addition-curing silicone does not adhere to smooth surfaces. Moreover, it has a high elasticity. It is well compatible and toxicologically harmless. Silicone shows a long life time.

According to an embodiment, silicone is a soft silicone. Alternatively, silicone can be a hard silicone. A hard silicone can have a high tear-strength. It can be stable and/or can have a long life time. The structured section can be configured such that the structured section comprising a hard silicone can have a soft wearing comfort.

In an embodiment, the polyurethane is a two-component polyurethane system. A structured section comprising a polyurethane, in particular a two-component polyurethane system, can be stiff at room temperature and can be soft at body temperature. This advantageously increases the wearing comfort. Additionally, such an ear piece can easily be inserted into an ear of a user.

In an embodiment, the contact section comprises a distal section and a proximal section, wherein the proximal section comprises the at least one structured section.

The proximal section can be the section of the contact section that can be inserted further into the ear canal of a user than the distal section, when the ear piece is used be the user as intended. In particular, the proximal section of the contact section is the section that can be close to the osseous part of the ear canal of the user (when the ear piece is used as intended). The proximal section can comprise the tip of the ear piece. The distal section can particularly be close to the cartilaginous part of the ear canal when the ear piece is used as intended.

According to an embodiment, the distal section adjoins the proximal section. According to the invention, the distal section can adjoin the non-contact section.

In an embodiment, the distal section and the proximal section are aligned to each other. According to an alternative embodiment, the distal section is tilted in comparison to the proximal section. A tilted arrangement of the proximal section related to the distal section can improve the fitting of the ear piece in the ear of a person when used as intended.

The at least one structured section of the proximal section can be configured such that it increases the convenience of the user when the ear piece is positioned in the user's ear as intended. According to an embodiment, the structured section of the proximal section is configured to exert a pressure on the user's ear that is lower than the pressure exerted by an ear pieces according to prior art. The structured section can be soft. According to an embodiment the structured section of the proximal section comprises a plurality of structure elements that form a plurality of lamellas.

According to an embodiment, the proximal section is configured to be easily inserted into the ear canal of the user. The proximal section can be configured for be comfortable to wear without pressure. Hence, the ear piece according to the invention can advantageously be used (i.e. be inserted in the ear canal) for a long time without giving pressure pain.

An embodiment is characterised in that the structured section comprises a ventilation system, wherein the ventilation system comprises at least one channel, in particular a plurality of channels. A channel can be formed by at least one structure element, for instance a groove.

An advantage of a ventilation system is the ventilation of the ear and/or a temperature control of the ear. Transpiration is advantageously reduced, when the ear piece is used as intended. Additionally, humidity can be transported away from the skin via the ventilation system.

According to another embodiment, the contact section comprises a distal section and a proximal section, wherein the distal section comprises the at least one structured section.

The at least one structured section of the distal section can be configured such that it increases the grip of the ear piece within the ear canal when the ear piece is in use as intended. In an embodiment, the distal section is configured to increase the adhesion of the ear piece in the ear canal when the ear piece is in use as intended. In particular, an inadvertent shift of the ear piece in the ear of the user is decreased, in particular prevented. Hence, advantageously the risk is decreased that the ear piece slips out of the user's ear. Advantageously, the risk is decreased to lose the ear piece. In other words this means that the wearing guarantee is advantageously increased.

According to an embodiment, the distal section is configured to plug or seal. In an embodiment, the distal section is configured to provide noise reduction.

An embodiment is characterised in that distal section comprises a first structured section and the proximal section comprises a second structured section, wherein the first structured section differs from the second structured section.

In an embodiment, the first structured section is configured to increase the holding of the ear piece, while the second structured section is configured to increases the convenience, e.g. by decreasing the pressure exerted on the ear canal when the ear piece is used as intended. Hence, advantageously the wearing comfort as well as the wearing guarantee is increased by a ear piece comprising the first and the second structured section.

In an embodiment, the at least one structure element is one of: a burl, a groove, a bar, a recess, or a cavity.

A structure element can extend along a longitudinal axis. It can extend along an extension direction. The structure element can have a cross section that extends perpendicular to the longitudinal axis. Along the extension direction, the shape and/or the size of the structure element can remain constant. Alternatively, along the extension direction, the shape and/or the size of the structure element can change.

A burl can have a circular cross section. In particular, a burl is directed outwards.

A groove can be directed inwards. A groove can have an elongated cross section that extends along an alignment direction. The alignment direction can extend parallel to the surface of the ear piece. The alignment direction can extend along the circumferential direction of the contact section. In an alternative embodiment, the groove extends along the extension direction of the ear piece, in particular the extension direction of the structured section. According to an alternative embodiment, the groove extends obliquely. This means that the alignment direction does neither run in parallel with the circumferential direction nor parallel with extension direction of the structured section.

The groove can comprise a plurality of groove segments. In particular, the groove can comprise a plurality of adjoining groove segments, wherein two neighbouring groove segments extend differently, i.e. have different alignment directions. For instance, by means of a plurality of groove segments, a groove extending in a zig-zag-pattern can be formed. In an embodiment, the groove and/or a groove segment can extend in a curved manner. By means of a plurality of adjoining curved groove segments, for instance, a groove extending in a wavelike manner can be formed.

A bar can have an elongated cross section. The bar can be directed outwards. A bar can have an elongated cross section that extends along an alignment direction. The alignment direction can extend along the circumferential direction of the contact section. In an alternative embodiment, the bar extends along the extension direction of the ear piece, in particular the extension direction of the structured section. According to an alternative embodiment, the bar extends obliquely. This means that the alignment direction does neither run in parallel with the circumferential direction nor parallel with extension direction of the structured section.

The bar can comprise a plurality of bar segments. In particular, the bar can comprise a plurality of adjoining bar segments, wherein two neighbouring bar segments extend differently, i.e. have different alignment directions. For instance, by means of a plurality of bar segments, a bar extending in a zig-zag-pattern can be formed. In an embodiment, the bar and/or a bar segment can extend in a curved manner. By means of a plurality of adjoining curved bar segments, for instance, a bar extending in a wavelike manner can be formed.

A recess can be directed inwards. The recess can have any shape.

A cavity can be directed inwards. The cavity can have a circular cross section. A cavity can be a hole. The cavity can be a depression, in particular a hollow depression.

According to an embodiment, the structured section comprises a plurality of structure elements that are arranged such that they form a particular pattern.

An embodiment is characterised in that the structured section is formed by a plurality of equal structure elements.

In an alternative embodiment, the structured section comprises a plurality of structure elements, wherein one structure element differs in at least one property from at least one other structure element of the plurality of structure elements. The at least one property can be the size, the height, the width, the length, the shape, the alignment direction and/or the orientation of the structure element.

The structure elements of the plurality of structure elements can be scattered (i.e. distributed unevenly). Alternatively, the structure elements of the plurality of structure elements can be distributed evenly.

According to an embodiment, the structured section is a homogenous structured section. This particularly means that the structured section consists of equal structure elements that are evenly distributed, wherein the alignment directions of the structure elements are aligned to one another. In an embodiment, the alignment directions of the structure elements extend in parallel.

The term "heterogeneous structured section" means that the structured section comprises a structure element that differs in at least one property from at least one other structure element of the plurality of structure elements and/or that the structure elements of the plurality of structure elements are scattered.

According to an embodiment the structure elements of the structured section are configured and arranged such that they form a pattern mimicking a tyre tread.

In an embodiment, the structure elements of the structured section are configured and arranged such that they form a plurality of lamella.

In another embodiment, the at least one structure element has a height and/or a width and/or a length of at least 25 µm.

The length of the structure element can be the extension of the structure element parallel to the central axis of the contact section.

The width of the structure element can be the extension of the structure element along the circumferential direction of the contact section.

The height can be the extension of the structure element perpendicular to the central axis of the contact section. The height can be the extension of the structure element along the extension direction of the structure element. The height specifies how far the structure element sticks out of the volume or how far the structure element recesses the volume.

In an embodiment, two neighbouring structure elements are distant to each other. The space between can be at least 25 µm. In an embodiment, there is a space between two neighbouring structure elements that have the same orientation (i.e. that both are directed inwards or that both are directed outwards).

According to an alternative embodiment, two neighbouring structure elements adjoin each other. In an embodiment, two neighbouring structure elements that have different orientations adjoin each other.

According to an embodiment, the ear piece extends along an extension direction, wherein the at least one structure element extends along the extension direction.

In other words this means that the alignment direction of the structure element extends parallel to the extension direction of the ear piece. In an embodiment, the alignment direction extends parallel to the extension direction of the contact section.

In an embodiment, the ear piece extends along a circumferential direction, wherein the at least one structured element extends along the circumferential direction.

In other words this means that the alignment direction of the structure element extends parallel to the circumferential direction of the ear piece. In an embodiment, the alignment direction of the structure element extends parallel to the circumferential direction of the contact section.

According to an embodiment, the structured section encloses the ear piece along the circumferential direction.

In an embodiment, the structure element encloses the contact section (and/or the ear piece) along the circumferential direction.

An embodiment is characterised in that the contact section consists of one piece.

Hence, at least the contact section can be produced in one production step. No additional subsequent production steps are necessary such the generation of the contact section is easy and time effective.

According to an embodiment, the ear piece consists of one piece.

Advantageously, the ear piece consists of one piece, wherein the ear piece can be produced in one production step such that no further production steps are needed for the generation. This can provide an easy and fast production of an ear piece according to the invention.

In another embodiment, the ear piece comprises or consists of a silicone, in particular an addition-curing silicone, or a polyurethane, in particular a two-component polyurethane system.

The silicone can be an addition-curing silicone. An addition curing silicone can vulcanise, i.e. can crosslink, without the release of a cleavage product. The advantage of such a silicone is that it hardly shrinks after and/or during the crosslinking process. This implies that it almost remains its size. In particular, the silicone remains its size and shape. The addition-curing silicone does not adhere to smooth surfaces. Moreover, it is has a high elasticity. It is well compatible and toxicologically harmless, such that it can be in contact with a human body for a long time without adverse effects. This means that the user can wear the ear piece over a long period of time without worrying about inadvertent side effects. Silicone shows a long life time.

According to an embodiment, silicone is a soft silicone. Alternatively, silicone can be a hard silicone. A hard silicone can have a high tear-strength. It can be stable and/or can have a long life time. The structured section can be configured such that the structured section comprising a hard silicone can have a soft wearing comfort.

In an embodiment, the polyurethane is a two-component polyurethane system. A structured section comprising a polyurethane, in particular a two-component polyurethane system, can be stiff at room temperature and can be soft at body temperature. This advantageously increases the wearing comfort. Additionally, such an ear piece can easily be inserted into an ear of a user.

In the following, further features, advantages and embodiments of the present invention are explained with reference to the Figures, wherein
- Fig. 1: shows a sectional front view of a casting form of an earpiece comprising an outwards directed texturing element,
- Fig. 2: shows a sectional front view of a casting form of an earpiece comprising an inwards directed texturing element,
- Fig. 3: shows a sectional front view of a casting form of an earpiece comprising a plurality of texturing elements,
- Fig. 4: shows a sectional front view of a casting form of a part of an earpiece comprising a plurality of texturing elements,
- Fig. 5: shows an ear piece comprising a structure element sticking out of the volume,
- Fig. 6: shows an ear piece comprising a structure element recessing the volume,
- Fig. 7: shows an ear piece comprising a non-contact section comprising a structure element,
- Fig. 8: shows an ear piece comprising a plurality of structure elements,
- Fig. 9: shows an ear piece comprising a plurality of structure elements,
- Fig. 10: shows a cross sectional view of an ear piece with equally spaced structure elements,
- Fig. 11: shows a cross sectional view of an ear piece,
- Fig. 12: shows a frontal view of an ear piece with a structure element in the distal section,
- Fig. 13: shows a frontal view of an ear piece with a structure element in the proximal section, and
- Fig. 14: shows a frontal view of an ear piece with a first structured section in the proximal section and a second structured section on the distal section.

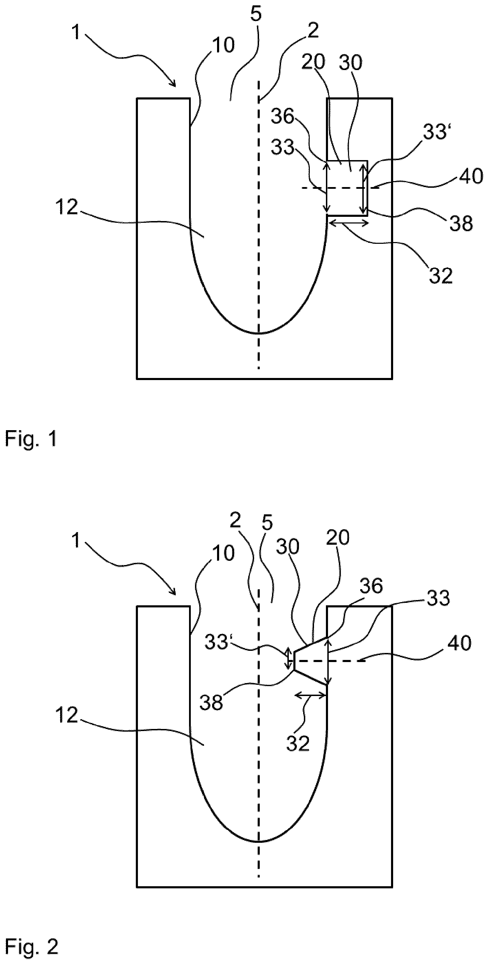

Figs 1 to 3 show a casting form 1 for the generation of an ear piece 100. The inner surface 10 delimits an inner volume 12. The inner volume 12 can be accessible via an opening 5. The inner volume 12 extends along a central axis 2. The different casting forms 1 presented in Fig. 1 and Fig. 2 each comprise a profiled section 20, wherein each profiled section 20 comprises each one texturing element 30.

In an embodiment, the texturing element 30 protrudes away from the central axis (Fig. 1). Alternatively, the texturing element 30 protrudes towards the central axis (Fig. 2).

A texturing element 30 can extend in an extension direction 40 (Fig. 1, Fig. 2). Each texturing element 30 can have a particular height 32, length 33 and width. Along the extension direction 40 of the texturing element 30, the length 33 and/or the width of the respective texturing element 30 can remain constant (see e.g. Fig. 1). This means that the length 33 at the foot 36 of the texturing element 30 equals the length 33' at the head 38 of the texturing element and the width at the foot 36 of the texturing element 30 equals the width at the head 38 of the texturing element. At the foot 36, the texturing element 30 arises from the inner surface 10. The head 38 is distant to the inner surface 10 surrounding the texturing element 30.

Alternatively, the length 33 and/or the width can change along the extension direction 40. In an embodiment the length 33 and/or the width change such that at the length 33 at the foot 36 of the texturing element 30 differs from the length 33' at the head 38 of the texturing element (see Fig. 2) and/or the width at the foot 36 of the texturing element 30 differs from the width at the head 38 of the texturing element. The texturing element 30 which is exemplarily shown in Fig. 2 tapers towards its head 38.

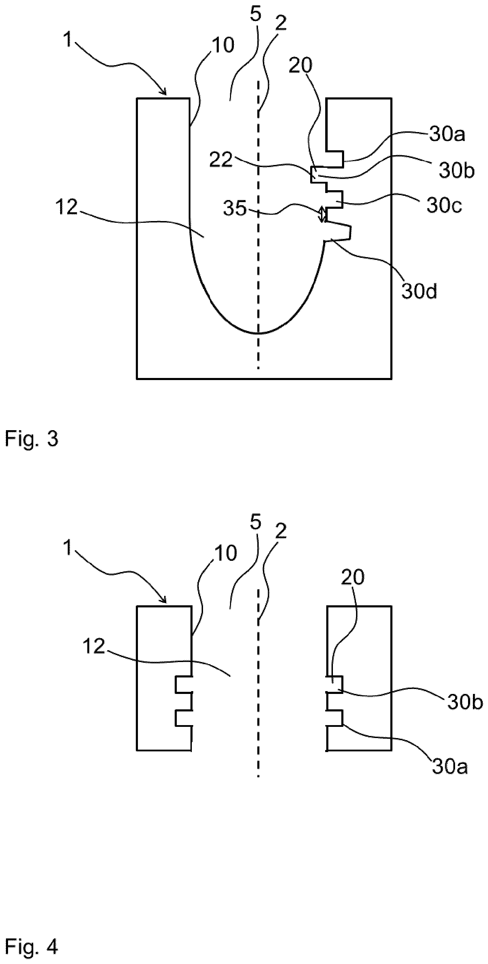

In Fig. 3, an embodiment of a casting form 1 is illustrated that comprises a profiled section 20 comprising a plurality of texturing elements 30a, 30b, 30c, 30d. The texturing elements 30a, 30b, 30c, 30d can differ in size and/or shape and/or orientation.

The texturing elements 30a, 30b, 30c, 30d of the plurality of texturing elements 30a, 30b, 30c, 30d can be arranged such that they form a pattern 22. Two neighbouring texturing elements 30c, 30d can be positioned such that a gap 35 between the neighbouring texturing elements 30c, 30d exists. Alternatively, two neighbouring texturing elements 30a, 30b can adjoin each other.

In an alternative embodiment, each texturing element of the plurality of texturing elements has the same shape, the same size and the same orientation. In other words this means that the texturing elements can be equal.

Fig. 4 shows a casting form 1 for the generation of a part of an ear piece, wherein the casting form 1 is particularly configured for the generation of at least a part of the contact section of the ear piece. The casting form 1 can comprise a plurality of texturing elements 30a, 30b.

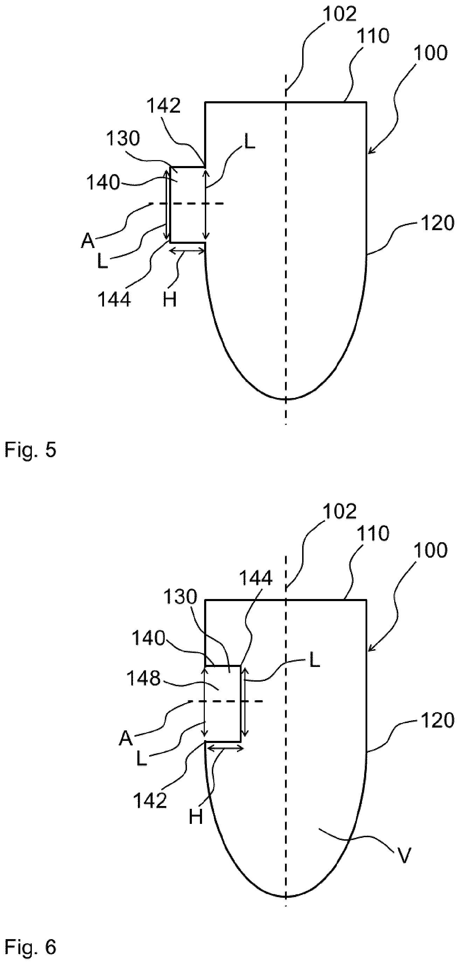

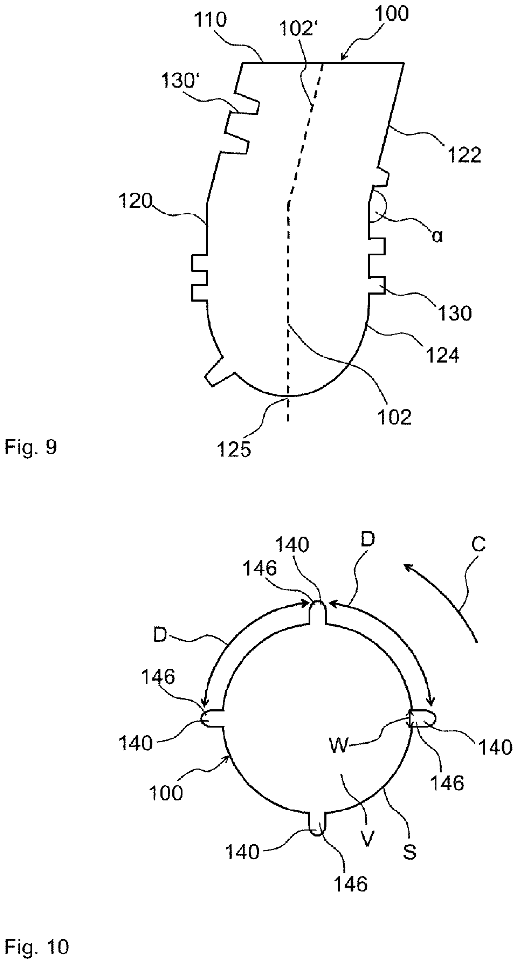

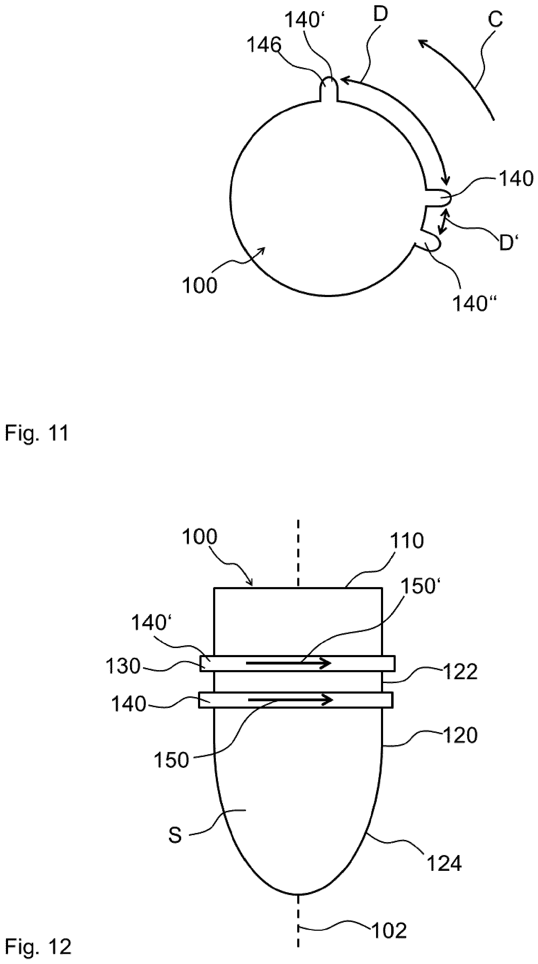

Fig. 5 - Fig. 14 show different embodiments of ear pieces 100, wherein Fig. 5 - Fig. 9 show frontal sectional views, Figs 10 and 11 show cross sectional views, and Figs 12 - 14 show frontal views. The ear piece 100 can extend along an extension direction 102. The ear piece 100 can comprise an outer surface 104.

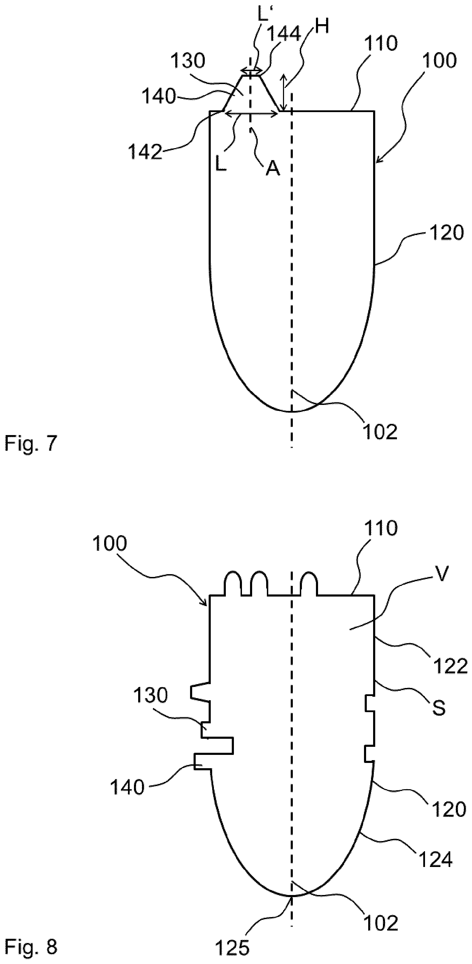

In an embodiment according to the invention, the contact section 120 of the ear piece 100 comprises a structured section 130 (Figs 5, 6). In Fig. 7, an ear piece 100 is illustrated that comprises a contact section 120 that is comprised in the non-contact section 110.

The structured section 130 can comprise at least one structure element 140. The structure element 140 can extend along a longitudinal axis A.

The structure element 140 can be configured such that it sticks out of the ear piece 100. In other words this means that the structure element 140 is configured as a protuberance 146 of the ear piece 100 (Figs 5, 7).

Alternatively, the structure element 140 can be configured such that it recesses the volume V of the ear piece 100. This means that the structure element 140 can be configured as a recess 148 (Fig. 6).

The structure element 140 comprises a foot 142 and a head 144 of the structure element 140. At the foot 142, the structure element 140 arises from the outer surface 104. The head 144 can be distant to the foot 142 of the structure element 140, in particular along the longitudinal axis A. The distance from the foot 142 to the head 144 is also referred to as the height H of the structure element. In particular, the distance from the foot 142 to the head 144 in the direction of the normal of the surface 104 is also referred to as the height H.

In an embodiment, the structure element 140 is configured such that the length L of the structure element 140 remains equal along the extension along the longitudinal axis A (Figs. 5, 6). In an embodiment, the length L and the width remain constant along the extension along the longitudinal axis A.

According to the invention, an ear piece 100 can comprise a plurality of structured sections 130 (Figs 8, 9). The ear piece 100 can comprise a structured section 130 of the plurality of structured sections 130 in the contact section and another structured section 130' in the non-contact section 130' (Fig. 8). In an embodiment, the ear piece 100 comprises a plurality of structured sections 130 in the contact section 120 (Fig. 9).

The ear piece 100 can be configured such that the contact section 120 comprises a distal section 122 and a proximal section 124 (Figs 8, 9). The distal section 122 and the proximal section 124 can adjoin each other. The distal section 122 can adjoin the non-contact section 110. The proximal section 124 can comprise the tip 125 of the ear piece 100.

The distal section 122 and the proximal section 124 can be aligned to each other (Fig. 8). According to an alternative embodiment, the distal section 122 and the proximal section 124 can be arranged at an angle α to each other (Fig. 9). This meant that there can be a tilt between the distal section 122 and the proximal section 124. The tilt can improve the fitting of the ear piece 100 in the ear of a user.

The structured section 130' of the distal section 122 can differ from the structured section 130 of the proximal section 124.

Figs 10 and 11 show cross sectional views of an ear piece 100 according to the invention. The cross sectional views illustrate the arrangement of a plurality of structure elements 140 along the circumferential direction C of the ear piece 100.

Along a circumferential direction C of the ear piece 100, structure elements 140 can be arranged equally spaced (e. g. Fig 10). This means that the distance D between each two adjacent (i.e. neighbouring) structure elements 140 is equal.

In an alternative embodiment, along the circumferential direction C, the space D between two neighbouring structure elements 140, 140' differs from the space D' between two other neighbouring structure elements 140, 140".

Figs 12 to 14 show frontal views of an ear piece 100.

In Fig. 12, an embodiment is presented in that the distal section 122 comprises the structured section 130. The structured section 130 comprises a plurality of structure elements 140. The structure element 140 can be a bar that extends along the alignment direction 150. The alignment direction 150 can extend along the circumferential direction of the contact section 120. The shown exemplary proximal section 124 does not comprise a structured element.

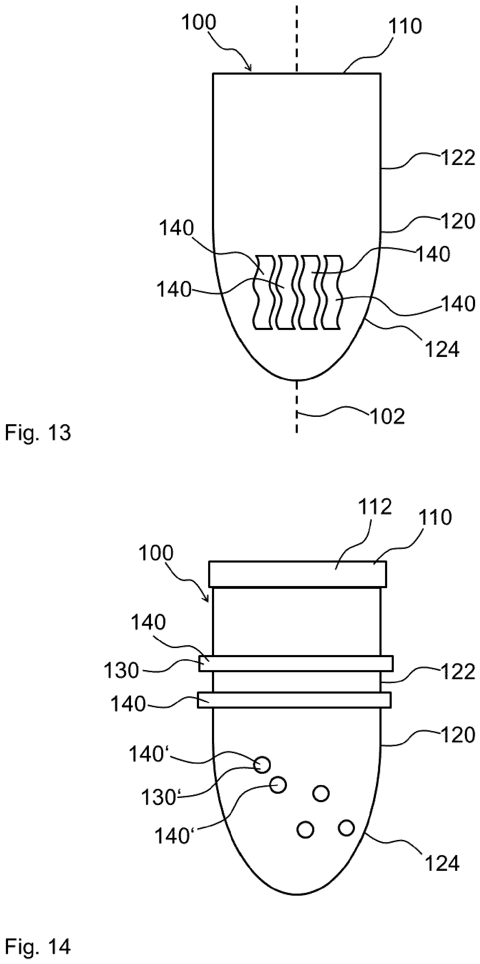

The ear piece 100 shown in Fig. 13 comprises the structured section 130 in the proximal section 124 of the contact section 120. The distal section 122 does not comprise a structured element.

In an alternative embodiment (Fig. 14), the ear piece 100 comprises a structured section 130 in the distal section 122 as well as another structured section 130' in the proximal section 124.

In an embodiment, the non-contact section 110 comprises the faceplate 112.

## Claims

1. A casting form (1) for the generation of an ear piece (100) or a part of an ear piece (100), wherein the ear piece (100) comprises a contact section (120) and a non-contact section (110),
**characterised in that**
the casting form (1) comprises an inner surface (10) delimiting an inner volume (12) for receiving a casting material, wherein the inner volume (12) is at least a part of the contact section (120),
wherein the inner surface (10) comprises at least one profiled section (20) comprising at least one texturing element (30).

2. Casting form (1) according to claim 1, **characterised in that** the inner volume (12) extends along a central axis (2), wherein the at least one texturing element (30) protrudes towards the central axis (2) into the inner volume (12) or wherein the at least one texturing element (30) protrudes away from the central axis (2).

3. Casting form (1) according to one of the claims 1 or 2, **characterised in that** the at least one texturing element (30) has a height (32) and/or a width and/or a length (33) of at least 25 µm.

4. Casting form (1) according to one of the claims 1 to 3, **characterised in that** the at least one profiled section (20) comprises or consists of a material for 3D printing, in particular a cured photoreactive material for 3D printing.

5. A method for the generation of a casting form (1) for the generation of an ear piece (100) or a part of an ear piece (100) according a to one of the claims 1 to 4, comprising the step of
- printing at least the at least one profiled section (20) by a 3D printer, in particular a printer configured for image projection-based method or laser-based printing.

6. An ear piece (100) for positioning in an ear of a person, wherein the ear piece (100) comprises a non-contact section (110) and a contact section (120), in particular generated by use of the casting form (1) for the generation of an ear piece (100) or a part of an ear piece (100) according to one of the claims 1 to 4,
**characterised in that**
the contact section (120) comprises at least one structured section (130) comprising at least one structure element (140), or
the contact section (120) and the non-contact section (110) each comprise at least one structured section (130) each comprising at least one structure element (140), or
the non-contact section (110) comprises at least one structured section (130) comprising at least one structure element (140), wherein the non-contact section (110) consists of one piece and comprises or consists of a silicone or a polyurethan.

7. Ear piece (100) according to claim 6, **characterised in that** the contact section (120) comprises a distal section (122) and a proximal section (124), wherein the proximal section (124) comprises the at least one structured section (130).

8. Ear piece (100) according to one of the claims 6 or 7, **characterised in that** the contact section (120) comprises a distal section (122) and a proximal section (124), wherein the distal section (122) comprises the at least one structured section (130).

9. Ear piece (100) according to one of the claims 7 or 8, **characterised in that** the distal section (122) comprises a first structured section (130) and the proximal section (124) comprises a second structured section (130'), wherein the first structured section (130) differs from the second structured section (130').

10. Ear piece (100) according to one of the claims 6 to 9, **characterised in that** the at least one structure element (140) is one of: a burl, a groove, a bar, a recess, or a cavity.

11. Ear piece (100) according to one of the claims 6 to 10, **characterised in that** the at least one structure element (140) has a height (H) and/or a width (W) and/or a length (L) of at least 25 µm.

12. Ear piece (100) according to one of the claims 6 to 11, **characterised in that** the ear piece (100) extends along an extension direction (102), wherein the at least one structure element (130) extends along the extension direction (102) of the ear piece (100).

13. Ear piece (100) according to one of the claims 6 to 12, **characterised in that** the ear piece (100) extends along a circumferential direction (C), wherein the at least one structured element (130) extends along the circumferential direction (C).

14. Ear piece (100) according to one of the claims 6 to 13, **characterised in that** the contact section (120) consists of one piece.

15. Ear piece (100) according to one of the claims 6 to 14, **characterised in that** the ear piece (100) comprises or consists of a silicone, in particular an addition-curing silicone, or a polyurethane, in particular a two-component polyurethane system.
